(19) Europäisches Patentamt
European Patent Office
Office européen des brevets

(11) Veröffentlichungsnummer: **0 242 559**
**A2**

(12) **EUROPÄISCHE PATENTANMELDUNG**

(21) Anmeldenummer: 87103389.0

(22) Anmeldetag: 10.03.87

(51) Int. Cl.⁴: **A61K 31/195** , A61K 31/395 , A61K 31/40 , A61K 31/445 , A61K 31/55

(30) Priorität: 15.03.86 DE 3608726

(43) Veröffentlichungstag der Anmeldung: 28.10.87 Patentblatt 87/44

(84) Benannte Vertragsstaaten: AT BE CH DE ES FR GB GR IT LI LU NL SE

(71) Anmelder: **HOECHST AKTIENGESELLSCHAFT**
**Postfach 80 03 20**
**D-6230 Frankfurt am Main 80(DE)**

Anmelder: **Max-Planck-Gesellschaft zur Förderung der Wissenschaften e.V.**
**Bunsenstrasse 10**
**D-3400 Göttingen(DE)**

(72) Erfinder: **Englert, Heinrich Christian, Dr.**
**Stormstrasse 13**
**D-6238 Hofheim am Taunus(DE)**
Erfinder: **Hropot, Max, Dr.**
**Friedrich-Stolz-Strasse 13**
**D-6093 Flörsheim am Main(DE)**
Erfinder: **Lang, Hans-Jochen, Dr.**
**Rüdersheimerstrasse 7**
**D-6238 Hofheim am Taunus(DE)**
Erfinder: **Greger, Rainer, Prof. Dr.**
**Im Bremmengässle 3**
**D-7843 Heitersheim(DE)**

(54) **Verwendung aminosubstituierter Benzoesäuren als Heilmittel gegen Diarrhoe und Arzneimittel auf Basis dieser Verbindungen.**

(57) Beschrieben wird die Verwendung aminosubstituierter Benzoesäurederivate der Formel I

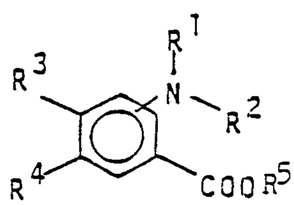

(I)

in welcher bedeuten:
$R^1$ und $R^2$ Wasserstoff, (Cyclo-) Alkyl, unsubstituiertes oder substituiertes Phenyl oder Naphthyl, oder $R^1$ und $R^2$ gemeinsam eine Kette $-(CH_2)_m-$ mit m = 3 bis 6 oder gemeinsam eine Kette $-(CH=CH)_n-$ mit n gleich 2 oder 3; $R^3$ Wasserstoff, Halogen oder Alkyl; $R^4$ Wasserstoff, $NO_2$; $R^5$ Wasserstoff oder einen unter physiologischen Bedingungen abspaltbaren Rest;
zur Herstellung eines Heilmittels gegen Diarrhoe.

EP 0 242 559 A2

## Verwendung aminosubstituierter Benzoesäuren als Heilmittel gegen Diarrhoe und Arzneimittel auf Basis dieser Verbindungen

Die Erfindung betrifft die Verwendung aminosubstituierter Benzoesäurederivate der Formel I

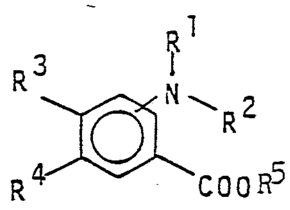

(I)

in welcher bedeuten:
$R^1$ und $R^2$, die gleich oder verschieden sind,
Wasserstoff,
$(C_1\text{-}C_6)$Alkyl, geradkettig oder verzweigt,
$(C_4\text{-}C_8)$-Cycloalkyl,
Ar, welches jeweils unsubstituiertes oder 1-3fach gleich oder verschieden durch $(C_1\text{-}C_2)$Alkyl, $(C_1\text{-}C_2)$-Alkoxy, F, Cl, Br, J, OH, $NH_2$, $C_6H_5$-NH, $CF_3$ substituiertes Phenyl oder Naphthyl bedeutet,
gemeinsam eine Kette $-(CH_2)_m$-mit m = 3 bis 6, die unsubstituiert oder durch 1-2 Methylgruppen substituiert ist, oder
gemeinsam eine Kette $-(CH=CH)_n$-mit n gleich 2 oder 3, die unsubstituiert oder mit 1 oder 2 Methylgruppen substituiert ist,
$R^3$ Wasserstoff, F, Cl, Br, J, $(C_1\text{-}C_6)$-Alkyl
$R^4$ Wasserstoff, $NO_2$
$R^5$ Wasserstoff oder einen unter physiologischen Bedingungen abspaltbaren Rest
und in welcher der Substituent $NR^1R^2$ in meta-oder ortho-Stellung zur Carboxylgruppe steht,
zur Herstellung eines Heilmittels gegen Diarrhoe.

Verbindungen der Formel I sind in vielen Fällen bereits bekannt. So werden beispielsweise Verbindungen I mit $R^3$, $R^4$,$R^5$ und $R^1$ = H, $R^2$ = Ar wobei Ar die obengenannte Bedeutung hat, als Antirheumatika und Antiphlogistika beschrieben (Arzneimittelforschung Drug Res. 33 (1), Nr. 4 a, 1983, 621 - 627). Am Beispiel der Meclophenaminsäure

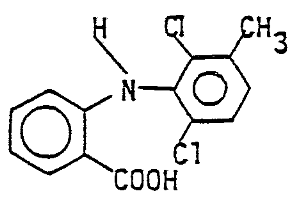

Meclophenaminsäure

konnte gezeigt werden, daß es als Nebenwirkung Diarrhoe auslöst (Ibid.: 631 - 635). Es war daher äußerst überraschend, daß Verbindungen der Formel I, insbesondere solche, die keine oder nur geringe antirheumatische Eigenschaften aufweisen ganz im Gegenteil zur Behandlung der Diarrhoe gut geeignet sind, insbesondere zur Behandlung solcher Diarrhoeformen, die durch bakterielle Toxine wie etwa das Choleratoxin ausgelöst werden.

Die Erfindung betrifft daher Arzneimittel, die Verbindungen der allgemeinen Formel I enthalten, und ihre Verwendung zur Behandlung der Diarrhoe.

Bevorzugt ist die Verwendung solcher Verbindungen der Formel I, in denen $R^1$ für cyclisches Alkyl mit 5 bis 7 Ringgliedern oder für Ar in der oben erwähnten Bedeutung, $R^2$ für H, $R^3$ für H, Alkyl mit 1-2 C-Atomen, Chlor oder Brom, $R^4$ für H oder $NO_2$ steht, $R^5$ für H und der Rest

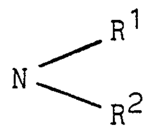

sich in ortho-Stellung zur Carboxygruppe befindet.

Bei Verwendung von Verbindungen mit $R^1/R^2$ in der Bedeutung von Ar beträgt die Gesamtzahl der C-Atome in $R^1$ und $R^2$ vorzugsweise bis zu 15, insbesondere bis zu 9.

Ebenfalls bevorzugt ist die Verwendung von Verbindungen I zur Bekämpfung der Diarrhoe, in welchen $R^1$ und $R^2$

a) eine gesättigte $-(CH_2)_m-$Kette mit m = 4 - 6 oder

b) eine doppelt ungesättigte $-(CH=CH)_n-$Kette mit n = 2 bilden,

$R^3$ für Alkyl mit 1-2 C-Atomen oder Chlor oder Brom stehen,

$R^4$ für Wasserstoff und $R^5$ für Wasserstoff steht und der Rest

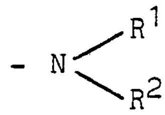

sich in meta-Stellung zur Carboxygruppe befindet.

Insbesondere folgende Verbindungen sind gut zur Behandlung der Diarrhoe geeignet:

1) 4'-Ethoxydiphenylamin-2-carbonsäure [3)]
2) 2-(1-Napthylamino)benzoesäure [3)]
3) 2'-Aminodiphenylamin-2-carbonsäure [3)]
4) 4'-Anilino-5-Nitro-diphenylamin-2-carbonsäure[3)]
5) 4'-Trifluormethyl-4-Nitro-diphenylamin-2-carbonsäure
6) 2-Cyclooctylamino-5-nitrobenzoesäure [4)]
7) 2-Cyclohexylamino-5-nitrobenzoesäure [4)]
8) Diphenylamin-2-carbonsäure [1)]
9) 4'-Methyldiphenylamin-2-carbonsäure [3)]
10) 4' -Chlordiphenylamin-2-carbonsäure [3)]
11) 4' -Nitrodiphenylamin-2-carbonsäure
12) 4' -Bromdiphenylamin-2-carbonsäure [4)]
13) 3', 4'-Dichlordiphenylamin-2-carbonsäure [1)]
14) 2'-Hydroxydiphenylamin-2-carbonsäure [3)]
15) 2'-Methoxydiphenylamin-2-carbonsäure [3)]
16) 5-Chlordiphenylamin-2-carbonsäure
17) 3',5-Dichlordiphenylamin-2-carbonsäure
18) 3'-Trifluormethyl-5-chlordiphenylamino-2-carbonsäure
19) 4-Methyl-3-N-pyrrolidinobenzoesäure
20) 4-Chlor-3-N-pyrrolidinobenzoesäure
21) 4-Chlor-3-N-pyrrolobenzoesäure
22) 4-Chlor-3-anilinobenzoesäure

<u>Literaturstellen:</u>

1) Arzneimittelforschung / Drug Res. 33 (1). Nr. 4a, 1983 621 - 627
2) Ibid.: 631 - 635
3) Liebigs Ann. Chem. 355, 312 - 348
4) Berichte 39, 1694

Herstellung der Verbindungen I

Soweit nicht bereits literaturbekannt, wurden die Verbindungen I nach folgenden Verfahren hergestellt:

a) Aus der 2-Chlor-bzw. 2-Brombenzoesäure der Formel II und einem Amin $ArNH_2$, wobei Ar die oben genannte Bedeutung aufweist unter Einwirkung von Cu-Pulver nach an sich bekannter Methodik [3]

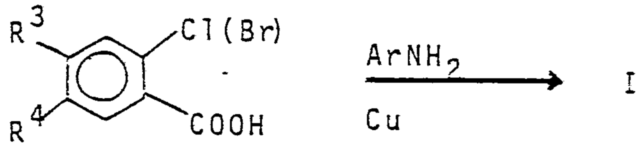


II

Folgende Verbindungen wurden so hergestellt:
Nr. 4, Schmp.: 232°C
Nr. 16, Schmp.: 204 - 206°C
Nr. 17, Schmp.: 200 - 203°C
Nr. 18, Schmp.: 212 - 214°C

b) Durch Austauschreaktion von Chlor (Brom) benzoesäuren II mit $R^4$ in der Bedeutung von $NO_2$ und einem Amin

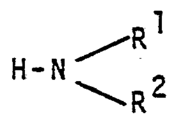

mit $R^1$, $R^2$ in der obengenannten Bedeutung ohne Cu-Katalyse in einem dipolar aprotischem Lösemittel wie Dimethylacetamid bei Temperaturen von 100 - 180°C
Folgende Verbindungen wurden hergestellt:
Nr. 4, Schmp.: 232°C
Nr. 5, Schmp.: 303 - 306°C als Na-Salz
Nr. 6, Schmp.: 194 - 196°C
Nr. 7, Schmp.: 186 - 189°C

c) Aus den 3-Aminobenzoesäuremethylestern III erhält man mit cyclischen Säureanhydriden IV Amide der Formel V

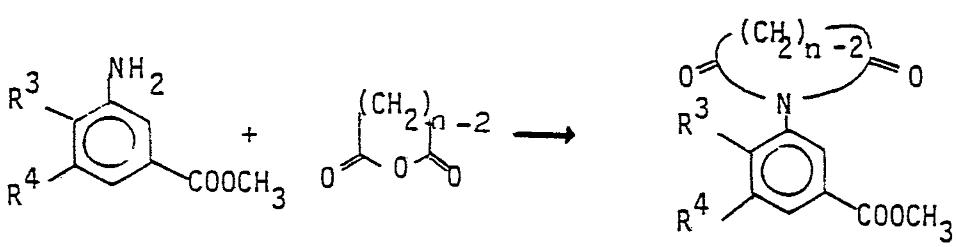

III IV V

mit m in der oben erwähnten Bedeutung. V kann mit $NaBH_4$ $BF_3 \bullet Et_2O$ in Diglyme zu VI reduziert werden:

V

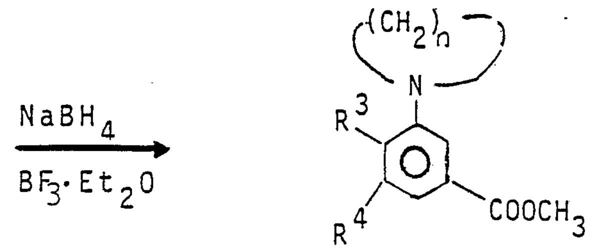


Die Ester VI werden nach Standardmethoden zu Verbindungen I hydrolysiert. Die Reaktionssequenz ist in der Literatur beschrieben (W. Merkel, D. Mania, D. Bormann, Liebigs Ann. Chem 1979, 461 - 469). Folgende Verbindungen wurden so hergestellt:

Nr. 19, Schmp.: 163 - 165°C

Nr. 20, Schmp.: 155 - 157°C

d) Aus 3-Brombenzoesäuremethylestern VII und Acetaniliden VIII unter Cu-Katalyse (Ullmann-Goldberg Reaktion) nach an sich bekannter Methodik (A.S. Freemann, J.R. Butter, L.D. Freedmann, J. Org. Chem. 1978, 43,4975 -4978).

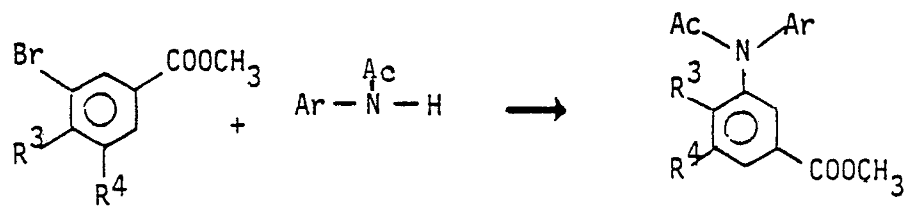

VII VIII IX

wobei Ar die oben erwähnte Bedeutung hat und Ac für die Acetylgruppe steht. Die Verbindungen IX werden nach Standardmethoden zu den Verbindungen I hydrolysiert.

Hergestellte Verbindungen:

Nr. 22, Schmp.: 223°C

e) Aus den 3-Aminobenzoesäureestern III und 2,5-Dimethoxytetrahydrofuranen erhält man Verbindungen X in an sich bekannter Weise (N.Elening, Clauson-kaas, Acta Chem. Scand. 6,876 (1952) ).

III +

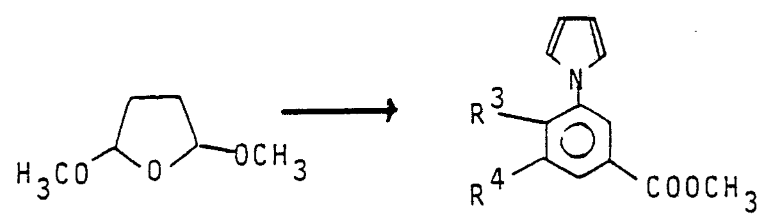

X

Verbindungen X werden in an sich bekannter Weise zu Verbindungen I hydrolysiert.

<u>Hergestellte Verbindung:</u>

Nr. 21, Schmp.: 222 - 223°C

Die erfindungsgemäß verwendeten Verbindungen der Formel I sowie deren pharmazeutisch verträgliche Salze - es kommen hier vor allem die Alkali-und Erdalkalisalze in Betracht, wie $Na^+$, $K^+$, $NH_4^+$ oder $Ca^{++}$ -Salze, aber auch Salze organischer Basen, Wie das Ethanolaminsalz sind von Bedeutung - sind Mittel zur Behandlung der Diarrhoe. Sie werden in Tagesdosierungen von mindestens 0,01 mg/kg, vorzugsweise 0,05 mg/kg, insbesondere 10 mg/kg bis maximal 200 mg/kg, vorzugsweise 50 mg/kg Körpergewicht und insbesondere 20 mg/kg, bezogen auf einen Erwachsenen von 75 kg Gewicht, in Kapseln, Dragees,

Tabletten oder Lösung allein oder in Kombination mit Elektrolytlösungen,die der Dehydration bei Diarrhoe entgegenwirken, enteral, z.B. oral, verabfolgt. Sie eignen sich zur Behandlung aller Krankheiten, bei denen es zu einem pathologisch gesteigerten Verlust von Wasser und Chlorid über den Darm kommt wie dies bei den verschiedensten Formen der Diarrhoe auftritt,vor allem bei der toxisch bedingten Diarrhoe infolge von Infektionskrankheiten wie etwa Cholera oder bei erblich bedingten Diarrhoeformen wie der kongenitalen Chloriddiarrhoe.

Die Tagesdosen werden in 1 bis 8, vorzugsweise 3-6 Einzeldosen verabreicht.

Die Verbindungen I werden entweder in reiner Form oder gemeinsam mit allgemein bekannten pharmazeutisch unbedenklichen Hilfsstoffen verwendet.

$R^5$ ist bevorzugt Wasserstoff, kann aber ebenfalls jede geeignete Gruppe sein, die unter physiologischen Bedingungen abgespalten wird und dabei die freie COOH-Gruppe bzw. deren Salze liefert.

## Ansprüche

1. Verwendung von Verbindungen der Formel I

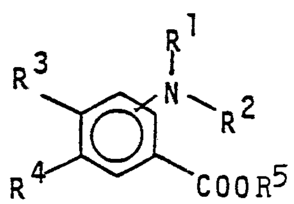

I

in welcher bedeuten:
$R^1$ und $R^2$, die gleich oder verschieden sind,
Wasserstoff,
$(C_1-C_6)$Alkyl, geradkettig oder verzweigt,
$(C_4-C_8)$-Cycloalkyl,
Ar, welches jeweils unsubstituiertes oder 1-3fach gleich oder verschieden durch $(C_1-C_2)$Alkyl, $(C_1-C_2)$-Alkoxy, F, Cl, Br, J, OH, $NH_2$, $C_6H_5$-NH, $CF_3$ substituiertes Phenyl oder Naphthyl bedeutet,
gemeinsam eine gesättigte
Kette $-(CH_2)_m$-mit m = 3 bis 6, die unsubstituiert oder durch 1-2 Methylgruppen substituiert ist, ist, oder gemeinsam eine Kette $-(CH=CH)_n$-mit n gleich 2 oder 3, die unsubstituiert oder mit 1 oder 2 Methylgruppen substituiert ist,
$R^3$ Wasserstoff, F, Cl, Br, J, $(C_1-C_6)$-Alkyl,
$R^4$ Wasserstoff, $NO_2$,
$R^5$ Wasserstoff oder einen unter physiologischen Bedingungen abspaltbaren Rest,
und in welcher der Substituent $NR^1R^2$ in meta-oder ortho-Stellung zur Carboxylgruppe steht,
zur Herstellung eines Heilmittels gegen Diarrhoe.

2. Verwendung nach Anspruch 1, dadurch gekennzeichnet, daß mindestens eine der folgenden Bedingungen erfüllt ist:
$R^5$ Wasserstoff,
$R^1$ $(C_5-C_7)$-Cycloalkyl, Ar wie in Anspruch 1 definiert
$R^2$ Wasserstoff
$R^3$ Wasserstoff, $(C_1-C_2)$-Alkyl, Cl, Br
$R^4$ Wasserstoff, $NO_2$
$NR^1R^2$ in ortho-Stellung zur Carboxylgruppe

3. Verwendung nach Anspruch 1 oder 2 dadurch gekennzeichnet, daß mindestens eine der folgenden Bedingungen erfüllt ist:
$R^1$ und $R^2$ gemeinsam a) eine $-(CH_2)$-Kette mit m gleich 4 bis 6, oder b) eine ungesättigte $-(CH=CH)_n$-Kette mit n gleich 2,
$R^3$ gleich $(C_1-C_2)$-Alkyl, Chlor, Brom,
$R^4$ gleich Wasserstoff und der Substituent $NR^1R^2$ in meta-Stellung zur Carboxylgruppe,
$R^5$ gleich Wasserstoff.

4. Verbindung I nach Anspruch 1 zur Verwendung als Mittel zur Behandlung der Diarrhoe.

5. Arzneimittel zur Behandlung der Diarrhoe, gekennzeichnet durch einen Gehalt an einer wirksamen Menge einer Verbindung I nach Anspruch 1, 2 oder 3 sowie pharmazeutisch unbedenkliche Hilfsstoffe.

6. Ausführungsform nach Anspruch 1, dadurch gekennzeichnet, daß die Gesamtzahl der C-Atome in $R^1/R^2$ in der Bedeutung Ar höchstens 15, vorzugsweise höchstens 9 beträgt.